# EUROPEAN PATENT SPECIFICATION

(11) **EP 0 633 240 B1**
(45) Date of publication and mention of the grant of the patent: **21.01.1998**
(21) Application number: 94109709.9
(22) Date of filing: 23.06.1994
(51) Int. Cl.: C07C 59/265, C07C 51/43

(54) **Recovery of citric acid from impure process streams by addition of strong acids and salts thereof**
Wiedergewinnung von Zitronensäure aus unreinen Prozessströmen durch Zusatz von starken Säuren und ihren Salze
Récupération d'acide citrique à partir de courants du procédé par l'addition d'acides forts et de leurs sels

(30) Priority: 06.07.1993 US 88219
(43) Date of publication of application: 11.01.1995
(73) Proprietor: HAARMANN & REIMER CORP., Springfield, New Jersey 07081 (US)
(72) Inventor: Felman, Steven W., Granger, IN 46530 (US); Patel, Chetna, Elkhart, IN 46514 (US); Patwardhan, Bhalchandra H., Elkhart, IN 46514 (US); Solow, David J., Elkhart, IN 46514 (US)
(74) Representative: Petrovicki, Wolfgang, Dr.

(56) References cited:
- DE-C- 627 445
- US-A- 4 851 573
- US-A- 5 032 686

## Description

Production of organic acids by fermentation is well known and is practiced on a large scale commercial basis throughout the world. In particular, citric acid is produced commercially primarily by an aerobic fermentation process, which employs molasses, starch, or a sugar such as glucose, fructose, sucrose, or converted lactose as a substrate and a fungus such as **Aspergillus niger** or yeast such as **Candida lipolytica** as biocatalyst. The fermentation product typically contains biomass from the spent microorganism, carbohydrates, amino acids, proteins and salts as well as citric acid which must be separated from the fermentation broth to provide a pure product. The invention described herein provides an improvement in the recovery of citric acid from impure process streams.

It can be difficult to separate the citric acid from the impurities generated during such a process. Two principal techniques are used at this time: solvent extraction as outlined in US Patent No. 4,275,234 to Baniel et al, and a lime-sulfuric acid process. The lime sulfuric acid process has the disadvantage of producing a vast quantity of calcium sulfate (gypsum) as a waste by-product which has to be disposed of by landfill. The latter is both expensive and environmentally distasteful. While the solvent extraction process is an improvement as it eliminates the gypsum waste product and improves process efficiency, it requires significant capital investment. Additional modifications of the solvent extraction process have been proposed in US Patent No. 4,994,609 to Baniel and Gonen. However, a large capital investment is still required. A modification of the lime-sulfuric process is proposed in EP 0 432 610 to Baniel and Eyal which provides for the concurrent production of citric acid and alkali citrates.

In addition, the literature describes many other techniques for the purification of the impure fermentation broth. Among the literature is published EP 0 167 957, assigned to Hoechst AG which discloses a process for isolating water soluble acidic compounds by bringing a solution of the acid into contact with a weakly basic, adsorbent, ion exchange resin, preferably those containing tertiary amino groups, and then desorbing the acid by water and/or steam.

*Offenlegungsschrift* DE 3 502 924, assigned to Benckiser GmbH, discloses a citric acid purification process involving membrane filtration, preferably ultrafiltration, together with adsorption on a non-ionic resin such as polystyrene or polyacrylamide followed by desorption and crystallization.

US Patent No. 4,851,573, to Kulprathipanja et al discloses a method for separation of citric acid from its fermentation broth by contacting the broth with a water-insoluble macroreticular gel of a weakly basic anionic exchange resin possessing tertiary amine groups in a cross-linked acrylic or stryene resin matrix. The citric acid is desorbed by water or dilute sulfuric acid.

US Patent No. 5,032,686, to Duflot et al and assigned to Roquette, Freres, discloses a method for the recovery of citric acid from a liquor containing the same, by successively: 1) putting the liquor containing the citric acid in contact with a cationic resin in the hydrogen form for a duration sufficient to reach an optimal adsorption (approximately 90%) of the acid; and 2) treating the resin by elution whereby the fraction of eluate rich in purified citric acid is recovered. A preferred eluant is water at a temperature higher than 40° C.

Hubertus Juetten, in his thesis entitled "The Enhanced Crystallization of Dicarboxylic Acids in Electrolyte Solutions," Michigan State University, 1992, states in the abstract that generally, carboxylic acid fermentations utilize anaerobic conditions and base addition for pH control. The salts of carboxylic acids then produced must be converted to the free acids and recovered. Juetten states that a process for the crystallization of carboxylic acids by means of salting-out with the electrolyte sulfuric acid has been developed. The work disclosed is primarily directed to the recovery of succinic acid from an aqueous solution of sodium succinate and sodium acetate. Juetten also discloses experiments on the salting out of citric acid, L-tartaric acid and DL malic acid from saturated solutions of pure acids in water by addition of sulfuric acid (page 25). No experiments were disclosed on the application of this process to fermentation broth. Juetten suggested that the salting out process could be used as a modification of the Berglund et al process (US Patent No. 5,034,105) which is described in the thesis, page 45, as a process for preparing a supersaturated solution of carboxylic acid from a fermentation broth. The anaerobic fermentation used to produce succinic acid operates optimally at pH's where salts of the organic acids rather than free acids are formed. Berglund provides an electrodialysis method whereby the mixed salt stream produced by such fermentation creates supersaturation in any system where the salt is more soluble than the acid. This process results in a mixture of two acids, succinic and acetic. Juetten provides a detailed proposal for the modification of the Berglund process with respect to the recovery of succinic acid from a mixture of sodium succinate and sodium acetate. There is no discussion or suggestion of the applicability of the modification to the recovery of an acid from an impure process stream in which a major constituent is not the salt of the acid of interest.

### SUMMARY OF THE INVENTION

The invention relates to a process for the recovery of citric acid from an impure aqueous solution of citric acid,
characterized in that a sufficient amount of a strong acid or a salt thereof selected from the group consisting of sulfuric acid, nitric acid, hydrochloric acid, hydroiodic acid, perchloric acid, chloric acid, hydrobromic acid, bromic acid, sodium chloride and sodium sulfate is added to the impure aqueous solution, thereby crystallizing the citric acid, and separating the crystallized citric acid from its mother liquor.

The claimed invention is directed to the recovery of citric acid from an impure solution by adding a sufficient amount of strong acid or salt to crystallize the citric acid and then separating the resulting crystals from the remaining mother liquor. This process avoids the complicated and expensive processes of the prior art. Moreover, as the level of impurities of the solution is continously increased if a continuous process is used, the difficulties with the attempted separation using prior art methods become increasingly more severe. There is nothing in prior art processes which would have taught one to expect that it would be possible to separate citric acid crystals from impure aqueous solutions including fermentation broths.

The preferred strong acid is sulfuric acid, and the recovery may be further improved by treating the mother liquor to obtain an additional yield of citric acid.
A preferred treatment is a resin treatment. Most preferably, the mother liquor is passed through an ion exclusion resin column. This further improvement comprises the additional steps of placing the liquor separated from the citric acid crystals onto a column and eluting with water. The impurities, including the strong acid or salt thereof, are eluted before the residual citric acid. When eluted, the residual citric acid obtained is added to the separated crystals, thereby further improving the yield. The augmented acid product may be further treated by additional crystallization to provide a more highly purified acid which may be used for foods or pharmaceuticals or may be further treated by spray granulation prior to packaging.

### DESCRIPTION OF THE INVENTION

Process of recovery of citric acid from fermentation broth is improved by treating the impure broth (sometimes referred to as fermentation "beer") to produce a concentrated solution of the citric acid. Addition of a strong acid or salt thereof causes crystallization of citric acid in high yield. It is not necessary or desirable to add a combination of salt (such as sodium sulfate) and sulfuric acid as is disclosed by Juetten in relation to succinic acid purification. Juetten reports yields of less than or equal to 80% for the recovery of succinic acid. However, the improved process of the invention is capable of providing yields above 80%.

After separation of the crystallized citric acid, the mother liquor containing large amounts of strong acid may be recycled into the process or can be run through a resin column to provide a waste stream of the added strong acid and impurities which may be discarded and a citric acid solution which may be added to the separated crystals for further purification.

This process is particularly useful for recovery of citric acid even though these fermentations do not result in a salt stream as discussed by Berglund and Juetten.

Residual liquors resulting from traditional citric acid recovery processes are commonly recycled back into the process. This recycling can build up significant levels of impurities. Since various process streams have different impurities contaminating the citric acid, over time, a poor quality of citric acid may result. The process provided herein is an improvement over present recovery processes, including the lime sulfuric process and solvent extraction process used to recovery citric acid commercially from fermentation beer, although it may also be used in conjunction with these recovery processes. In the improved process, the impurities, such as color, carbonizables, other acids such as gluconic acid, oxalic acid, phosphoric acid, hydrochloric acid, sulfuric acid and salts thereof which are left in the residual liquor, are readily removed. The improved process recovers more citric acid from a process stream than a simple recrystallization step and is basically a one step purification process. The improved process has several advantages over existing processes including: higher productivity, simplicity, reduction of process stream recycling, lower quantity of required reagents, decreased impurities in the recovery process and lower operating costs.

The recovery can be further improved and made even more economical by treating the liquor remaining after separation of citric acid crystals to obtain an additional yield, for example by a resin treatment of the residual liquor. The resin treatment used may be an ion exchange resin column or, preferably, an ion exclusion resin column. With this additional improvement, recovery can be increased to over 90%, making the process economically viable for large scale production of citric acid which is sold as a commodity and competes worldwide on a price basis. This additional improvement provides the further advantages of: higher productivity without the requirement of additional reagents; a simpler overall process than presently utilized recovery processes with fewer purification steps; a more flexible process not requiring regeneration; waste treatment reduction; with lower operating and maintenance costs.

### Citric Acid

The improved process of the invention is particularly useful for the recovery of citric acid from its fermentation broth. However, other organic acids, produced by fermentation at a pH which does not produce a salt stream may also be recovered with the process. Recovery processes related to such acids are considered equivalent to the citric acid process disclosed herein. In addition, although the improved process is particularly useful as applied to the recovery of citric acid produced by fermentation because of the large quantity of impurities encountered and the large scale operation usually involved, the process may also be applied to the recovery of other organic acids from any process stream.

### Strong Acids and Salts Thereof

Strong acids and salts thereof are a class of compounds (Fundamentals of Analytical Chemistry, 4th Ed., Skoog, Douglas and West, Donald, 1982, CBS College Publishing, Page 6) which ionize in a solvent to produce an electrically conducting medium. Strong acids and their salts ionize completely in the solvent. Particular examples of strong acids and salts thereof include, but are not limited to, sulfuric acid, nitric acid, hydrochloric acid, hydroiodic acid, perchloric acid, chloric acid, hydrobromic acid, bromic acid, sodium chloride and sodium sulfate; others are well known to those of skill in the art. For the process described herein, strong acids or salts chosen from the group consisting of sulfuric acid, hydrochloric acid, sodium sulfate, and nitric acid are preferred. Sulfuric acid is particularly preferred.

### Resin

Ion exchange resins, preferably ion exclusion resins, may be used to provide an additional improvement to the recovery of the desired citric acid. This invention, in contrast to the Duflot et al patent, separates citric acid from residual sulfuric acid. The appropriate process stream may be added to a resin column and water used to elute the acid of interest. In this process, the impurities are separated from the acid based on their affinity to the resin and size. Generally, colored impurities, salts and carbohydrates are eluted before the acid. With the preferred resin treatment, the capacity of the resin is very large and, unlike resin adsorption processes, no regeneration is required. An ion exclusion process is particularly beneficial since it is a continuous operation using fixed or moving columns. In contrast to typical recovery processes, for example lime-sulfuric or solvent extraction, the process of this invention may be used as a one step purification and does not require elaborate or expensive capital equipment.

### A Detailed Description of the Process Using Crystallization by Sulfuric Acid as an Example

The use of the improvements described above will be described in greater detail with respect to the recovery of citric acid directly from its fermentation broth by crystallization with sulfuric acid in order to provide guidance to one of skill in the art. The steps in the production process would therefore include:
1. fermenting an appropriate carbon and hydrogen source as a substrate in the presence of an appropriate microorganism to produce a fermentation broth containing citric acid together with impurities including the biomass residue of the microorganism;
2. treating the fermentation broth to substantially remove the biomass, thereby providing a partially purified fermentation broth containing citric acid, unreacted substrate, and other impurities;
3. treating the partially purified fermentation broth, from which the biomass has been removed, to produce a concentrated solution of citric acid;
4. adding a sufficient amount of sulfuric acid to the concentrated solution to crystalize citric acid; and
5. separating the citric acid crystals from the mother liquor.

The partially purified fermentation broth is preferably concentrated by evaporation to within about 10% of the saturation point of citric acid, most preferably to about the saturation point of citric acid.

The amount of sulfuric acid added is determined by the amount of the water in the fermentation broth in question. One of skill in the art will be able to adjust the amount depending on the particular conditions and impurities in the concentrated solution. The separated citric acid crystals are commonly washed and may be further treated by dissolution and recrystallization prior to packaging or by dissolution and introduction into spray granulation equipment prior to packaging.

Since the competition in the production of citric acid is so intense and so price competitive, it is preferable to increase the yield of citric acid by further treating the mother liquor which was separated from the crystallized citric acid by addition of sulfuric acid. Treatment by passing it through a resin column is preferred, but it may also be treated by any other method to obtain an additional yield. Most preferred is treatment with an ion exclusion resin column. Preferred ion exclusion resins are of the class of strong acidic cation-exchange resins such as sulfonated polystyrene and divinylbenzene copolymers in the proton form. The column is eluted with water at ambient temperature. The initial eluate contains sulfuric acid and other impurities such as colorants and carbohydrates. This initial eluate may be combined with the water removed when the fermentation broth is evaporated to dilute the sulfuric acid and the combined waste stream may be treated and discarded. The second eluate, containing citric acid, may be used to dissolve the separated citric acid crystals and then the resulting solution may be treated for final recovery by crystallization or spray granulation.

The addition of strong acids or salts thereof may also be used in conjunction with other recovery processes such as solvent extraction or lime sulfuric. For example, a process stream produced by such recovery processes may be treated to obtain a concentrated solution of citric acid and treated by the addition of strong acid or salt. Alternatively a residual liquor, obtained after initial separation of citric acid from these recovery processes, may be treated directly by the addition of strong acid or salt if it is already sufficiently concentrated. When used with such residual liquors it is particularly surprising that the addition of sulfuric acid will crystallize citric acid leaving other impurities in solution.

The method of practicing the invention is further illustrated by the following examples.

### EXAMPLES

### Example 1: Recovery of Citric Acid by Crystallization with Sulfuric Acid.

To 100 ml of a saturated solution of citric acid obtained by treatment of fermentor broth at 50° C, 3 ml of sulfuric acid was added slowly. At this point, citric acid starts crystallizing. The mixture is stirred for two hours and gradually cooled to 30°C. This mixture was filtered through a Buchner funnel. Eighty-three percent (83%) of citric acid was recovered. Impurities such as color and carbonizables were reduced by ninety percent (90%).

To 100 ml of a saturated solution of citric acid obtained by evaporation of a process stream from a citric acid recovery at 55°C, 3 ml of sulfuric acid was added slowly. External cooling may also be used to prevent rapid rise of temperature of the solution. At this point, citric acid starts to crystallize rapidly. The mixture is stirred for two hours and gradually cooled to 30° C. The mixture is then filtered through a Buchner funnel. Ninety-two percent (92%) of citric acid was recovered. Impurities such as carbonizables and color were reduced by 90%

### EXAMPLE 2: Ion Exclusion Step

The resin treatment may be applied most advantageously to recover the citric acid left in the mother liquor of Example 1 to improve the overall yield of citric acid.

A column of 1.5 x 100 cm was packed with Bayer Lewatit MDS 1368 ion exclusion resin in the proton form. The mother liquor, after separation of citric acid from Example 1, was used as a feed to the column. The feed was loaded on the column and water was used as the eluant. The pulse feed was 0.5 bed volume (BV) and the flow rate was 2.0 ml/min. Seventy fractions were collected on the fraction collector. It took one bed volume of water for elution and another bed volume for rinse for one pulse feed. The fractions were analyzed for sulfates and citric acid by high pressure liquid chromatography (HPLC). The sulfates elute earlier than citric acid.

It should be understood that many modifications and variations can be made in the proportions and components used herein without departing from the spirit and scope of the inventions, which is solely defined by the claims.

## Claims

1. A process for the recovery of citric acid from an impure aqueous solution of citric acid,
characterized in that a sufficient amount of a strong acid or a salt thereof selected from the group consisting of
sulfuric acid, nitric acid, hydrochloric acid, hydroiodic acid, perchloric acid, chloric acid, hydrobromic acid, bromic acid, sodium chloride and sodium sulfate
is added to the impure aqueous solution, thereby crystallizing the citric acid, and separating the crystallized citric acid from its mother liquor.

2. The process of claim 1, wherein the impure aqueous solution is a fermentation broth.

3. The process of claim 1, wherein the impure aqueous solution is the residual liquor obtained after initial separation of citric acid from a solvent extraction or a lime sulfuric acid process.

4. The process of claim 2, wherein the biomass residue of microorganism is removed from the fermentation broth prior to the additon of strong acid or salt thereof.

5. The process of claims 1 to 4 in which the mother liquor is passed through an ion exchange resin to recover an additional yield of citric acid.

6. The process of claims 1 to 5 in which the strong acid or salt thereof is selected from the group consisting of sulfuric acid, hydrochloric acid, sodium chloride, sodium sulfate, and nitric acid.

7. The process of claims 1 to 5 in which the strong acid is sulfuric acid.

## Patentansprüche

1. Verfahren zur Gewinnung von Zitronensäure aus einer verunreinigten wäßrigen Lösung von Zitronensäure, dadurch gekennzeichnet, daß eine ausreichende Menge einer starken Säure oder eines Salzes davon, die bzw. das aus der Gruppe ausgewählt ist, die aus Schwefelsäure, Salpetersäure, Chlorwasserstoffsäure, Iodwasserstoffsäure, Perchlorsäure, Chlorsäure, Bromwasserstoffsäure, Bromsäure, Natriumchlorid und Natriumsulfat besteht, zu der verunreinigten wäßrigen Lösung gegeben wird, wodurch die Zitronensäure auskristallisiert, und die kristallisierte Zitronensäure von ihrer Mutterlauge abgetrennt wird.

2. Verfahren gemäß Anspruch 1, wobei die verunreinigte wäßrige Lösung eine Fermentationsflüssigkeit ist.

3. Verfahren gemäß Anspruch 1, wobei es sich bei der verunreinigten wäßrigen Lösung um den Rückstand handelt, den man nach der ersten Abtrennung von Zitronensäure aus einem Lösungsmittelextraktions- oder Kalk-Schwefelsäure-Verfahren erhält.

4. Verfahren gemäß Anspruch 2, wobei der Biomassenrückstand des Mikroorganismus aus der Fermentationsflüssigkeit entfernt wird, bevor man die starke Säure oder das Salz davon zugibt.

5. Verfahren gemäß Anspruch 1 bis 4, wobei die Mutterlauge durch ein Ionentauscherharz geleitet wird, um eine zusätzliche Fraktion Zitronensäure zu gewinnen.

6. Verfahren gemäß Anspruch 1 bis 5, wobei die starke Säure oder das Salz davon aus der Gruppe ausgewählt ist, die aus Schwefelsäure, Chlorwasserstoffsäure, Natriumchlorid, Natriumsulfat und Salpetersäure besteht.

7. Verfahren gemäß Anspruch 1 bis 5, wobei es sich bei der starken Säure um Schwefelsäure handelt.

## Revendications

1. Procédé de récupération de l'acide citrique à partir d'une solution aqueuse impure d'acide citrique,
caractérisé en ce qu'une quantité suffisante d'un acide fort ou d'un sel de celui-ci choisi parmi le groupe consistant en
l'acide sulfurique, l'acide nitrique, l'acide chlorhydrique, l'acide iodhydrique, l'acide perchlorique, l'acide chlorique, l'acide bromhydrique, l'acide bromique, le chlorure de sodium et le sulfate de sodium,
est ajoutée à la solution aqueuse impure, faisant ainsi cristalliser l'acide citrique et séparant l'acide citrique cristallisé de sa liqueur mère.

2. Procédé selon la revendication 1, dans lequel la solution aqueuse impure est un bouillon de fermentation.

3. Procédé selon la revendication 1, dans lequel la solution aqueuse impure est la liqueur résiduelle obtenue après séparation initiale de l'acide citrique à partir d'un procédé d'extraction par solvant ou d'un procédé à l'acide sulfocalcique.

4. Procédé selon la revendication 2, dans lequel le résidu de la biomasse des microorganismes est éliminé du bouillon de fermentation avant l'addition d'acide fort ou de sels de celui-ci.

5. Procédé selon l'une quelconque des revendications 1 à 4, dans lequel la liqueur mère est passée à travers une résine échangeuse d'ions pour donner un rendement supplémentaire en acide citrique.

6. Procédé selon l'une quelconque des revendications 1 à 5, dans lequel l'acide fort ou son sel est choisi parmi le groupe consistant en l'acide sulfurique, l'acide chlorhydrique, le chlorure de sodium, le sulfate de sodium et l'acide nitrique.

7. Procédé selon l'une quelconque des revendications 1 à 5, dans lequel l'acide fort est l'acide sulfurique.
